# EUROPEAN PATENT SPECIFICATION

(11) **EP 2 510 936 B1**
(45) Date of publication and mention of the grant of the patent: **13.04.2016**
(21) Application number: 12170747.5
(22) Date of filing: 27.09.2006
(51) Int. Cl.: A61K 38/00, A61K 38/03, A61K 38/08, A61K 38/10, C07K 7/00, A61K 47/48, C07K 7/06, C07K 7/08, C07K 14/00

(54) **Calcium binding peptides**
Calciumbindende Peptide
Peptides de liaison du calcium

(30) Priority: 28.09.2005 US 722071 P
(43) Date of publication of application: 17.10.2012
(62) Divisional of application: 06804228.2
(73) Proprietor: The Regents of the University of California, Oakland, CA 94607 (US)
(72) Inventor: Yarbrough, Daniel, Los Angeles, CA 90039 (US); Shi, Wenyuan, Los Angeles, CA 90045 (US); Hagerman, Elizabeth, Beverly Hills, CA 90210 (US); Tetradis, Sotirios, Culver City, CA 90232 (US); Qi, Fengxia, Laguna Niguel, CA 92677 (US); He, Jian, Los Angeles, CA 90034 (US); Rutherford, Bruce, Seattle, WA 98109 (US); Eckert, Randal, Ellensburg, WA 98926 (US); Wu, Ben, San Marino, CA 91108 (US)
(74) Representative: Zwicker, Jörk

(56) References cited:
- WO-A1-01/97834
- WO-A1-2005/089826
- WO-A2-03/093314
- US-A1- 2003 064 498
- VEIS ARTHUR ET AL: "Properties of the (DSS)n triplet repeat domain of rat dentin phosphophoryn", EUROPEAN JOURNAL OF ORAL SCIENCES, MUNKSGAARD INTERNATIONAL PUBLISHERS, COPENHAGEN, DK, vol. 106, no. 1 Suppl, 1 January 1998 (1998-01-01), pages 234-238, XP009128411, ISSN: 0909-8836
- HUNTER G K ET AL: "NUCLEATION OF HYDROXYAPATITE BY BONE SIALOPROTEIN", PROCEEDINGS OF THE NATIONAL ACADEMY OF SCIENCES OF USA, NATIONAL ACADEMY OF SCIENCE, WASHINGTON, DC, US, vol. 90, 1 January 1993 (1993-01-01), pages 8562-8566, XP002923936, ISSN: 0027-8424
- GEORGE ANNE ET AL: "The carboxyl-terminal domain of phosphophoryn contains unique extended triplet amino acid repeat sequences forming ordered carboxyl-phosphate interaction ridges that may be essential in the biomineralization process", JOURNAL OF BIOLOGICAL CHEMISTRY, vol. 271, no. 51, 1996, pages 32869-32873, XP002565022, ISSN: 0021-9258
- YARBROUGH ET AL: "Specific binding and mineralization of calcified surfaces by small peptides", CALCIF TISSUE INT, vol. 86, 1 December 2009 (2009-12-01), pages 58-66, XP002565023,

## Description

### BACKGROUND

Compounds that bind specifically to calcified surfaces include small fluorescent molecules such as tetracycline, calcein, and alizarin, and large calcium-binding proteins such as dentin phosphoprotein (DPP, often referred to as phosphophoryn) and amelogenin, DPP is one of the major noncollagenous proteins found in the dentin extracellular matrix and has long been implicated in the nucleation of hydroxyapatite (HA) during dentin mineralization (Lee 1980; Lussi 1988; Veis 1998; Hao 2004). Human DPP is derived from proteolytic cleavage of dentin sialophosphoprotein (DSPP). Human DPP is a highly flexible (Cross 2005), highly phosphorylated (Lee 1980) protein consisting primarily of a large number of Asp-Ser-Ser amino acid repeats (Gu 2000). The biochemical characteristics of DPP have been extensively investigated. These investigations have revealed that immobilized DPP causes marked increases in the rate of hydroxyapatite nucleation, though this effect is not seen with DPP in solution or with dephosphorylated DPP. In addition, high concentrations of DPP have been shown to inhibit HA crystal growth (Lussi 1988; Veis 1998; Saito 2000). Also, Gorge et al. (J Biol. Chem. 1996 Doc 20;271 (51):32869-73) described triplet amino acid repeats of the C-terminal domain of phosphophoryn. US 6673900 B2 dislcoses a peptide comprising the sequence (DSS)4 and discusses the role of a related peptide for bone and tooth mineralization. US 2005/288299 A1 described peptides comprising a PG, GP, PI or IG amino acid motif and stimulating the development, maintenance and repair of bone, cartilage and associated connective tissue.

### SUMMARY

A composition is provided that includes one or more calcium binding peptides,. These calcium binding peptides comprise the three amino acid repeat sequence (X-Y-Z)ₙ, wherein X is aspartic acid, glutamic acid, asparagine, or glutamine, Y and Z alanine, serine, or threonine, and n is a number from 3 to 15, wherein said peptides are not phosphorylated and wherein said calcium binding peptides bind calcium phosphate. The calcium binding peptides have a length of up to 100 amino acids. In certain embodiments, n is a number from 2 to 8. In certain embodiments, X is aspartic acid and Y and Z are serine. In certain of these embodiments, the calcium binding peptides may have the amino acid sequence set forth in any of SEQ ID NOs:4-10.

in certain embodiments, the calcium binding peptides provided herein may be linked to a detectable marker, selected from the group consisting of a fluorophore, chromophore, affinity tag, antigen tag, radioactive label, and spin label, or an antimicrobial peptide. In certain embodiments, the conjugate or moiety may be linked to the calcium binding peptides via an amino acid linker.

In certain embodiments, methods are provided for treating a tooth defect characterized by tooth damineralization in a subject by administering a composition comprising the calcium binding peptides disclose herein. As disclosed herein, administration of these calcium binding peptides is capable of inducing remineralization of tooth surfaces.

In certain embodiments, methods are provided for treating a bone defect characterized by bone demineralization or decreased bone density in a subject by administering a composition comprising the calcium binding peptides disclosed herein. As disclosed herein, administration of these calcium binding peptides is capable of inducing remineralization of bone surfaces and increasing bone density.

In certain embodiments, methods are provided for identifying a tooth defect characterized by tooth demineralization in a subject by administering a composition comprising the calcium binding peptides disclosed herein, wherein the peptides are conjugated to a detectable marker, and then detecting this marker using either the naked eye or a detection device. As disclosed herein, these calcium binding peptides are capable of selectively or preferentially binding portions of the tooth exhibiting demineralization.

In certain embodiments, methods are provided for identifying a bone defect characterized by bone demineralization in a subject by administering a composition comprising the calcium binding peptides disclosed herein, wherein the peptides are conjugated to a detectable marker, and then detecting this marker using either the naked eye or a detection device. As disclosed herein, these calcium binding peptides are capable of selectively or preferentially binding portions of the bone exhibiting demineralization.

Further, methods are disclosed for identifying calcification in a subject in tissue other than bone or teeth by administering a composition comprising the calcium binding peptides disclosed herein, wherein the peptides are conjugated to a detectable marker, and then detecting this marker using either the naked eye or a detection device. As disclosed herein, these calcium binding peptides are capable of binding calcium and calcium oxalate. Calcifications that may be identified using this method include, for example, arterial plaque, kidney stones, and sesamoids. The peptides may be used to treat these inappropriate calcifications, for example by conjugating the peptides to a therapeutic moiety and using the peptides to target the therapeutic moiety to the calcification site.

In certain embodiments, compositions comprising the calcium binding peptides disclosed herein are provided for use in treating tooth defects characterized by tooth demineralization or bone defects characterized by bone demineralization. In certain embodiments, these compositions may be used to detect or diagnose tooth or bone defects characterized by demineralization.

Further, kits are disclosed that contain a composition comprising the calcium binding peptides disclosed herein. The kits may include instructions for use or administration. These kits may be used for treating tooth defects characterized by tooth demineralization or bone defects characterized by bone demineralization. These compositions may be used to detect or diagnose tooth or bone defects characterized by demineralization.

### BRIEF DESCRIPTION OF THE DRAWING

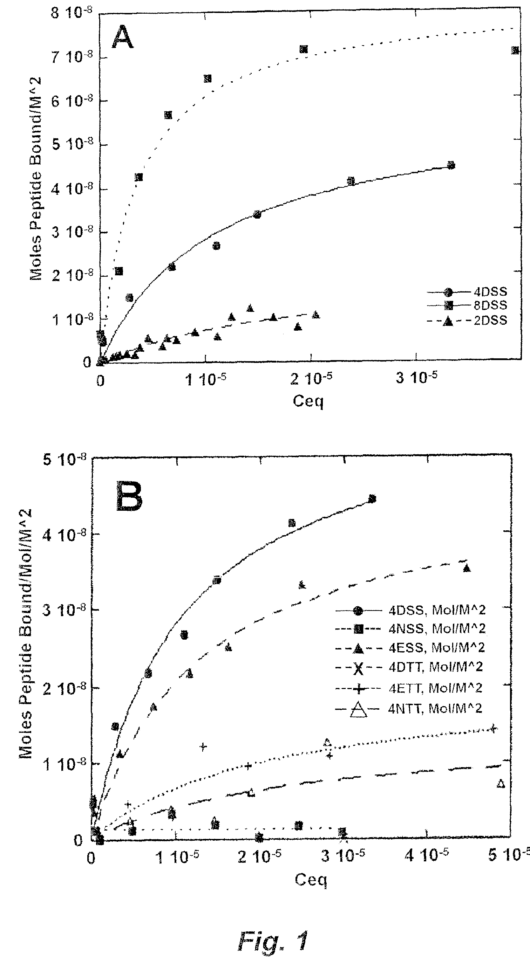

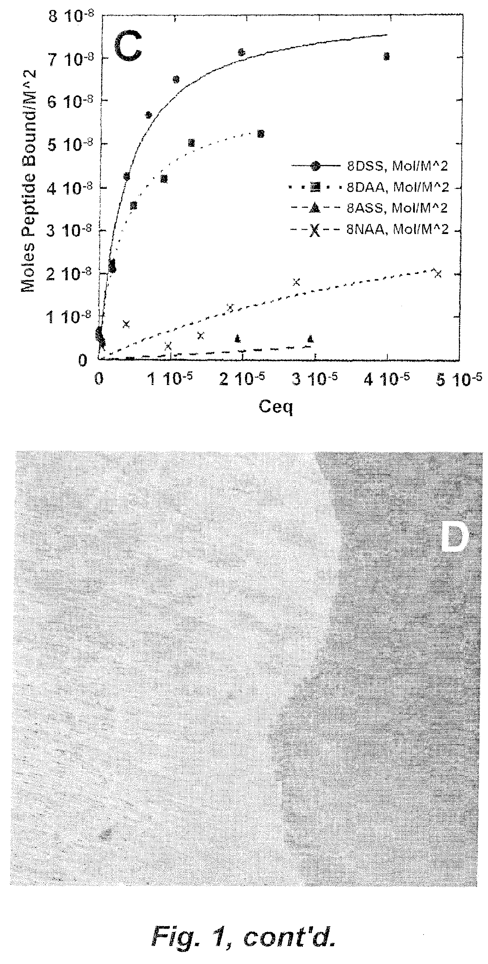

Figure 1: Binding affinities of DSS and DSS variant peptides for hydroxyapatite and tooth surfaces. A-C. Fluorescein-labeled peptides at concentrations ranging from 0-100 µM were incubated with 0.3 mg of hydroxyapatite nanocrystals (specific surface arena=100 m²/g) for ten minutes, followed by removal of the hydroxyapatite by centrifugation. The amount of peptide in the mixture before and after hydroxyapatite removal was determined by Abs₄₈₀. Isotherms were plotted and fit to the Langmuir equation to extract values for K_{A}, a constant reflecting the affinity of the peptide for the hydroxyapatite surface. D. Sagitally sectioned human teeth were incubated with 12.5 µM 5(6) carboxyfluorescein-labeled 6DSS peptide for ten minutes, follower by rinsing. Peptide binding to tooth surfaces was visualized by Confocal Laser Scanning Microscopy (CLSM) using blue laser illumination (*λ*=488nm) and a FITC emission filter. Left side, light colored region: dentin. Right side, dark colored region: enamel.

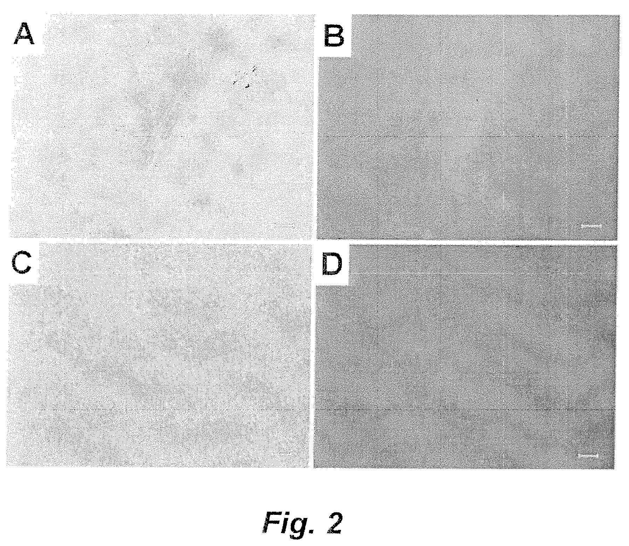

Figure 2: Binding of 6DSS peptide to mineralized mouse bone marrow nodules (MBMNs). Mouse bone marrow cultures were grown to confluence, then treated for three weeks with 2.5 µM scrambled Control peptide or 2.5 µM 6DSS. Cultures were imaged by fluorescence microscopy using a FITC filter set. A. Brightfield image of mineralized MBMNs from a culture treated with 6DSS. B. Fluorescence image of the field shown in (A). The strong staining (light coloration) of the central nodule mass indicates binding by the fluorescently labeled 6DSS peptide. C. Brightfield image of mineralized MBMNs from a culture treated with scrambled control peptides. D. Fluorescence image of the field shown in (C), illustrating both the lack of binding of the control peptide to the MBMN's and the lack of autofluorescence within the samples.

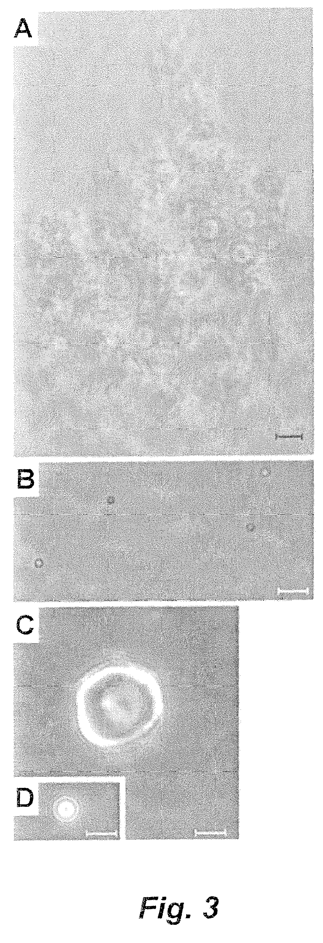

Figure 3: Interaction of immobilized 8DSS peptide with CaHPO4. Streptavidin-coated polystyrene beads were incubated with biotin-conjugated 8DSS (A and C) or unconjugated biotin (B) and (D). Beads were washed and incubated in a solution of PBS+ 1mM CaCl₂ +1 mM NaHPO₄ for twelve days prior to imaging. A. Brightfield micrographs of aggregates of amorphous calcium phosphate that accumulated around DSS-coated beads. All calcium phosphate aggregates of significant size were associated with one or more beads, B. Brightfield image of representative biotin-blocked beads (no DSS peptide). No significant amount of precipitate was associated with these beads. C. Phase-contrast micrograph of a DSS-coated bead with a more ordered accretion of calcium phosphate around its exterior (note the spherical center of the object). These objects were not seen in control samples (biotin-blocked, no DOSS peptide), as illustrated in (D). Scale Bars= 4 µm.

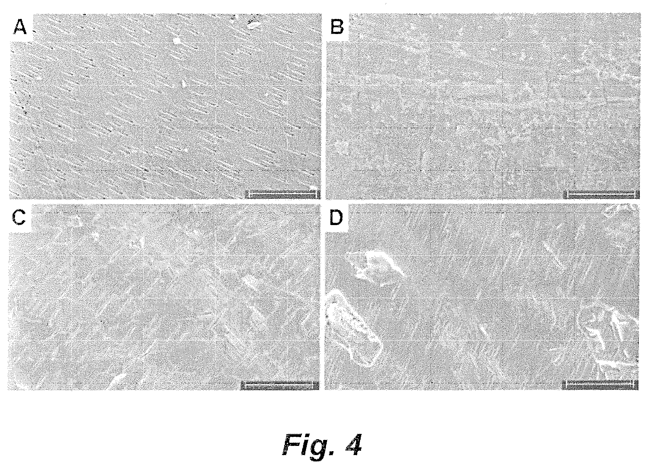

Figure 4: Remineralization of tooth surfaces with DSS peptides. Extracted human teeth were sagitally sectioned and demineralized with 19% EDTA gel for 1 hour, followed by immersion in deionized water and ultrasonication to remove excess debris. Samples were treated as indicated, then imaged by Scanning Electron Microscopy. Scale bars=50 *µ*m. A. Demineralized control samples. B. Treated with 8DSS for 1 hour, rinsed, and demineralized using Quell Desensitizer. C. Treated with buffer only, remineralized using Quell Desensitizer. D. No treatment, remineralized using Quell Desensitizer.

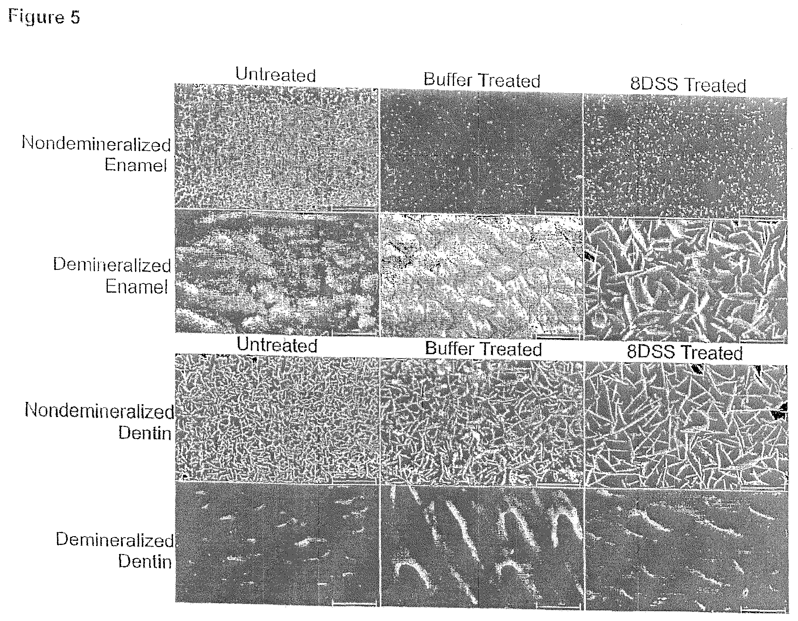

Figure 5: Nucleation of hydroxyapatite on enamel (top) and dentin (bottom) surfaces. Surfaces were prepared and treated as described in Example 6 and as indicated by the labels, followed by imaging using Scanning Electron Microscopy. The upper group (top two rows) represents enamel samples, while the lower group (bottom two rows) represents dentin samples. Within each group, the top row represents samples that were not demineralized prior to treatment, and the bottom row represents samples that were demineralized by treatment with phosphoric acid. Scanning Electron Micrographs are shown, scale bars=10 µM. Left column: samples that were not exposed to any treatment prior to nucleation and crystal growth. Center column: samples that were exposed to buffer only prior to nucleation and crystal growth. Right column: samples that were exposed to 12.5 µM 8DSS peptide prior to nucleation and crystal growth. Crystal growth indicates early nucleation.

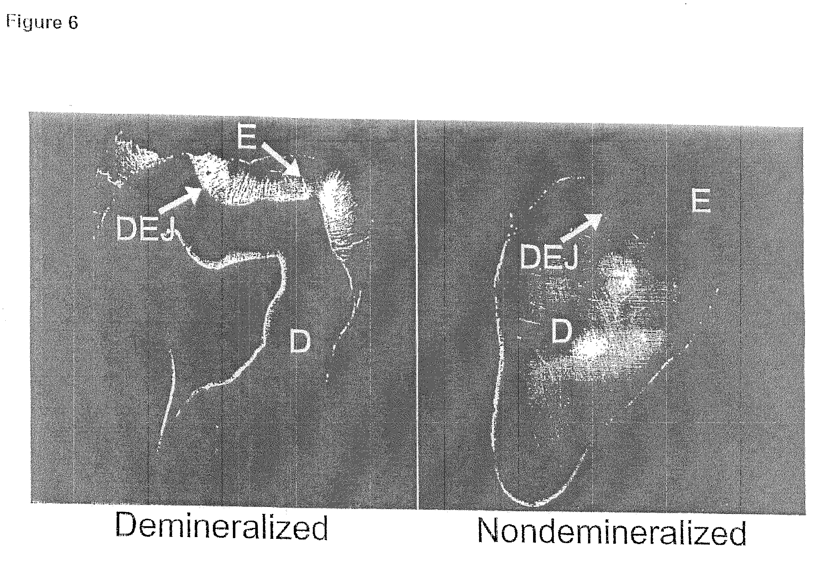

Figure 6: Tissue specificity of 8DSS peptide binding and its dependence on mineralization state. Demineralize and nondemineralized human teeth samples were incubated with 12.5 µM 5(6)-carboxyfluorescein-labeled 8DSS peptide as described in Example 9, and sections were rinsed and imaged by CLSM. The left panel shows the binding pattern of 8DSS peptide to demineralized tissue. Primary binding is to the demineralize enamel (E) with little binding to the dentin, (D). The opposite pattern is observed in the nondemineralized sample, where primary binding is to the dentin (D) with little or no binding to the enamel (E). The dentin-enamel junction, labeled DEJ, is clearly demarcated in both cases.

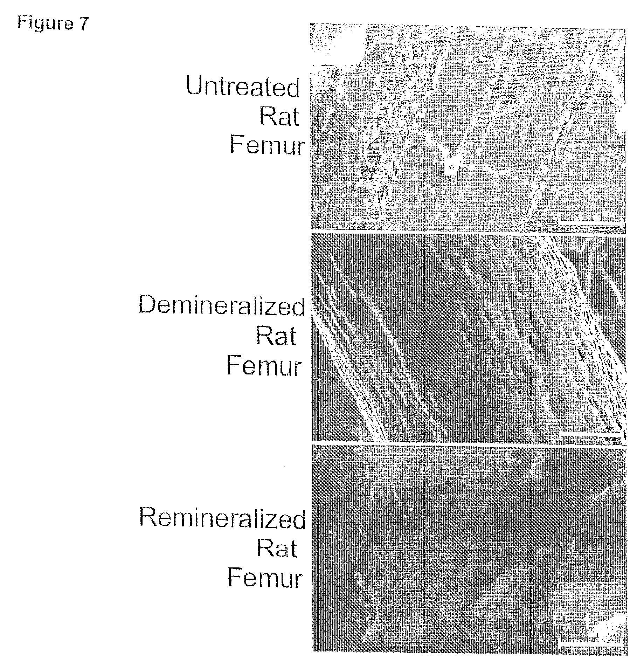

Figure 7: Mineralization of bone. Rat femurs were obtained from sacrificed animals under a shared tissue protocol. Samples were demineralized, rinsed, and ultrasonicated to remove debris. Test samples were treated with 8DSS peptide for one hour, rinsed, remineralized using Quell Desensitizer as described in Example 8, then imaged by Scanning Electron Microscopy. SEM images are shown. Top: untreated sample, showing the surface of the bone completely covered by mineral. Center: demineralize sample, showing Haversian canals exposed by removal of the mineral layer. Bottom: Bone treated with 8DSS and Quell Desensitizer (an aqueous CaCl₂/K₂HPO₄ solution), showing reestablishment of the mineral layer.

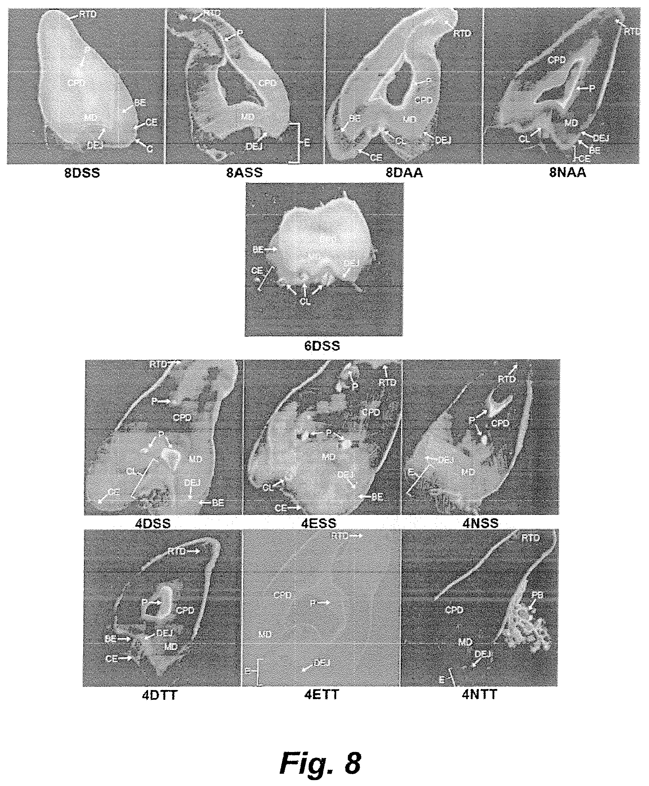

Figure 8: Fluorescence micrographs showing tissue specificity in the binding of DSS peptides and variants. Adult human teeth were exposed to 12.5 µM 5(6)-carboxyfluorescein-labeled peptide without demineralization, washed expensively, and imaged by CLSM. For each section, multiple scans were collected and assembled in an automated mode to generate panels of images representing an area of 13x13 mm, sufficient in most cases to encompass the whole section. The peptides used for each section are labeled beneath each panel, and within each pane! the tooth is oriented with the root toward the top of the image and the crown toward the bottom. Tissue layers are labeled as follows: RTD= Root Tip Dentin: CPD= Circumpulpal Dentin; MD= Mantle Dentin: P= Pulp Cavity Wall; DEJ= Dentin-Enamel Junction; E= Enamel; BE= Basal Enamel; CE= Cortical Enamel; CL= Carious Lesion; PB= Periodontal Bone.

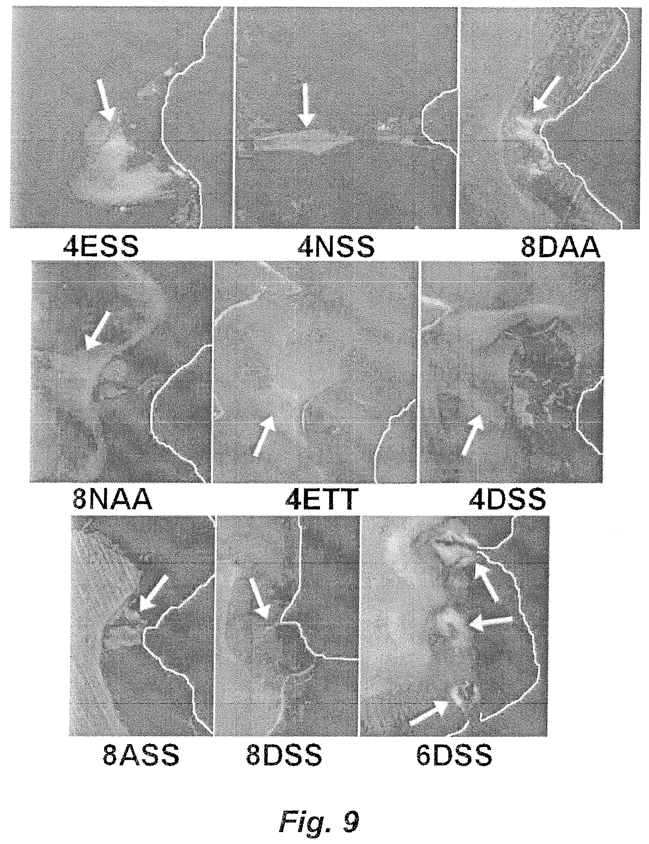

Figure 9: Fluorescence micrographs showing specific binding of DSS peptides and variants to carious lesions in teeth. Adult human teeth were exposed to 12.5 uM 5(6)-carboxyfluorescein-labeled peptide without demineralization, washed extensively, and imaged by CLSM. Microscope and camera settings were optimized separately for each sample. Each panel shows a region of the tooth section encompassing a carious lesion. White traces on the right side of each panel identify the position of the tooth surface, while arrows indicate the position of the stained lesion.

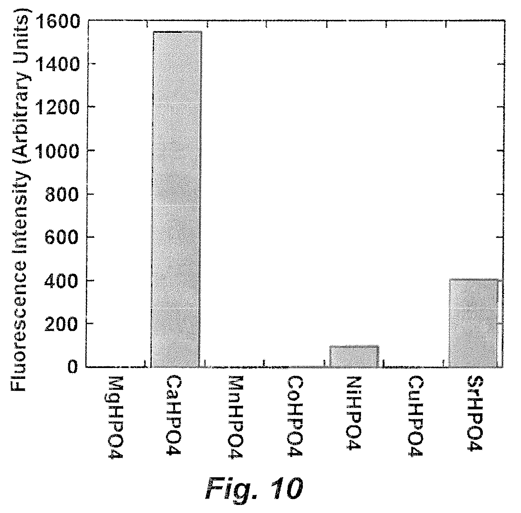

Figure 10; Relative levels of 8DSS peptide binding to various inorganic phosphate precipitates. 100 mM sodium phosphate (pH 7.5) was combined with 100 mM solutions of MgCl₂, CaCl₂, MnCl₂, CoCl₂, NiSO₄, CuSO₄, and SrCl₂, respectively. Suspensions were made of each phosphate salt at a concentration of approximately 0.5% (w/v) in the presence of 12.5 µM 5(6)-carboxyfluorescein-labeled 8DSS peptide. After a ten minute incubation at room temperature, samples were washed and imaged by fluorescence microscopy. The intensity of the fluorescent staining of the inorganic phosphate particles in each sample was assessed by measuring the pixel intensity values using the GIMP (www.gimp.org) as described in Example 11. Normalized fluorescence values are plotted here. On the X-axis, the various inorganic phosphate precipitates are identified. On the Y-axis, the relative fluorescence intensity values are plotted.

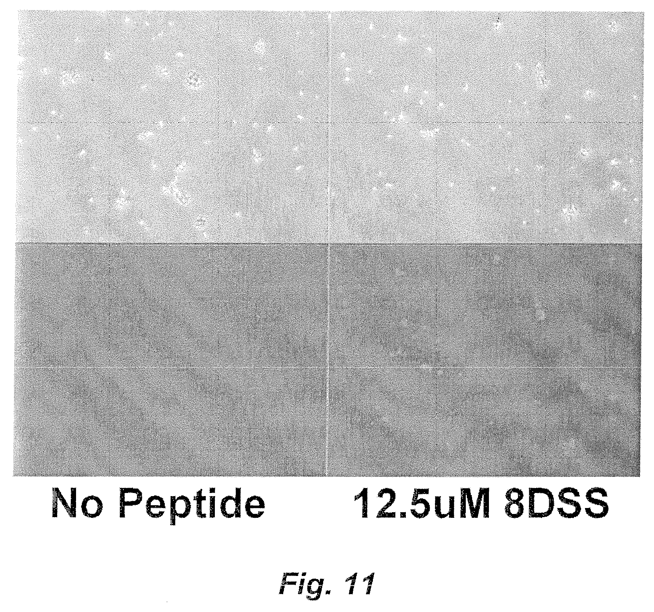

Figure 11: Binding of 8DSS peptide to calcium oxalate. Calcium oxalate crystals were exposed to 12.5 µM 8DSS peptide, washed, and imaged by fluorescence microscopy as described in Example 12. The left side of the figure shows unstained calcium oxalate crystals. The brightfield image (top) confirms the presence of crystals, while the fluorescence image of the same region (bottom) confirms that calcium oxalate has no visible Fluorescence under these conditions. The right side of the figure shows calcium oxalate crystals stained with 5(6)-carboxyfluorescein-labeled 8DSS peptide. The brightfield image (top) confirms the presence of crystals, and the fluorescence image of the same region (bottom) shows bright staining of the crystals by the labeled peptide.

### DETAILED DESCRIPTION

The following description of the invention is merely intended to illustrate the invention. As such, the specific modifications discussed are not to be construed as limitations on the scope of the invention, It will be apparent to one skilled in the art that various equivalents, changes, and modifications may be made without departing from the scope of the invention, and it is understood that such equivalent embodiments are to be included herein,

### Abbreviations

The following abbreviations are used herein: BE, basal enamel; CE, cortical enamel; CL, carious lesion; CLSM, confocal laser scanning microscopy; CPD, circumpulpal dentin; D, dentin; DEJ, dentin-enamel junction; DPP, dentin phosphoprotein; E, enamel; DSPP, dentin sialophosphoprotein; HA. hydroxyapatite; MD, mantle dentin; MIC, minimum inhibitory concentration; P, pulp cavity wall; PB, periodontal bone; SEM, scanning electron microscopy; RTD, root tip dentin; *λ*, excitation wavelength.

Amino acids are abbreviated using the standard system set forth below.

| **Amino acid** | **One letter abbreviation** | **Three letter abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamic acid | E | Glu |
| Glutamine | Q | Gln |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Phosphoserine | S_{P} | Sep |
| Proline | P | Pro |
| Serine | S | Ser |
| Threonine | T | Thr |
| Tryptophan | W | Trp |
| Tyrosino | Y | Tyr |
| Valine | V | Val |

Unless indicated otherwise by a "D" prefix, e.g., D-Ala, the stereochemistry of the alpha-carbon of the amino acids and aminoacyl residues in peptides described herein is the natural or "L" configuration.

### Calcium binding peptides

Compounds currently available for the direct remineralization of decalcified tissues consist mainly of various formulations of free or protein-bound calcium phosphate and/or sodium fluoride. Enhancement of calcification in biological tissues is generally achieved by manipulating cell signaling in bone and tooth precursor cells, or by increasing the global calcium concentration using calcium-fortified foods, dietary supplements, or other treatments rich in free or protein-bound calcium. Previous studies have described a formulation that recruits calcium phosphate to the tooth surface to enhance remineralization (see U.S. Pat. No. 6,780,844). However, enhancement of calcification by directly and specifically targeting calcium to surfaces has not been demonstrated.

Disclosed herein are a series of small calcium binding peptides made up of variations of the Asp-Ser-Ser motif found in DPP. These peptides have been shown to bind tightly and specifically to calcium phosphate surfaces. In addition, these peptides have been shown to recruit calcium phosphate to such surfaces and to serve as binding moieties for the attachment of fluorescent labels to calcified surfaces regardless of their phosphorylation state.

The peptides disclosed herein, referred to generally as DSS peptides, are composed of various numbers and/or combinations of the three amino acid Asp-Ser-Ser motif of DPP or variations thereof. Examples of three amino acid repeats that may be utilized include, but are not limited to, Asp-Ser-Ser (DSS, SEQ, ID NO. 1), Glu-Ser-Ser (ESS, SEQ, ID NO. 2), Asp-Thr-Thr (DTT, SEQ. ID NO. 3), Glu-Thr-Thr (ETT, SEQ, ID NO. 4), Asn-Ser-Ser (NSS, SEQ, ID NO. 5), Asn-Thr-Thr (NTT, SEQ, ID NO. 6), Gln-Ser-Ser (QSS, SEQ. ID NO. 7), Gln-Thr-Thr (QTT, SEQ. ID NO. 8), and variations thereof. Alternatively or in addition to these repeat sequences, the peptides disclosed herein may Include minor variations of these repeats, including but not limited to Asp-Ser-Thr (DST, SEQ. ID NO. 9), Asp-Ala-Ala (DAA, SEQ. ID NO. 10), or Ala-Ser-Thr (as, SEQ. ID NO. 11). One or more amino acid residues within a three amino acid repeat may be chemically modified. For example, the peptides may contain one or more Ser or Thr residues in which a hydroxyl group has been modified by the addition of a phosphate group. The peptides may vary in length from three to greater than fifty amino acids.

The binding affinity of the peptides disclosed herein for calcified surfaces may be controlled by altering the composition and number of repeats. For example, inclusion of one or more Asp-Ser-Ser (SEQ. ID NO. 1) repeats will increase the binding affinity of the peptide, because this sequence exhibits the highest affinity of any of the repeats tested, The binding affinity of the peptide may also be increased by increasing the number of three amino acid repeats. Peptides containing more than six repeats generally exhibit greater binding affinity than those with fewer repeats, the peptides disclosed herein may have a binding affinity (K_{A}) for hydroxyapatite of greater than 15,000 M⁻¹, this binding affinity may be greater than 50,000 M⁻¹, greater than 100,000 M⁻¹, greater than 200,000 M⁻¹, and greater than 300,000 M⁻¹.

The peptides may contain one or more additional amino acids that are not part of a three amino acid repeat sequence. For example, the repeat portion of the peptide may be fused to an amino acid sequence having an additional functionality, such as for example an antimicrobial peptide sequence such as the 2c-4, PL135, or b-34 peptide sequences. the repeat portion of the peptide may be fused to the additional amino acid sequence via a linker sequence, such as for example a triglycine sequence.

The peptides disclosed herein may comprise the sequence (X-Y-Z)ₙ, wherein X is an amino acid selected from aspartic acid, glutamic acid, asparagine, alanine and glutamine, Y and Z are amino acids selected from alanine, serine, threonine, phosphoserine, phosphothreonine, and their derivatives, and n is a number between 1 and 20. n may be between 1 and 15, between 1 and 10, and between 3 and 8.

The calcium binding peptides disclosed herein have been shown to induce calcium phosphate crystal growth on demineralized enamel and on both demineralized and nondemineralized dentin, depending on treatment conditions. Likewise, the peptides have been shown to induce remineralization of bone. Thus, compositions comprising the calcium binding peptides disclosed herein may be used to enhance mineralization by recruiting free-floating calcium phosphate particles to calcified surfaces. These peptides may bind to calcified surfaces and/or free-floating calcium phosphate aggregates. Concurrent binding of calcified surfaces and free-floating aggregates results in increased calcium phosphate concentration near the calcified surface, which leads to enhanced remineralization of the surface. By modulating the size and binding affinity of the peptides, it is possible to alter the amount of calcium bound to the surface. Remineralization of teeth can results in complete or partial occlusion of dentinal tubules.

Compositions comprising the calcium binding peptides disclosed herein may be used to remineralize a tooth, prevent or slow tooth daminaralization, treat tooth damage, defects, illnesses, or anomalies, form mineral layers at or below the surface of a tooth, alter the mineral density of a tooth, such as for example increasing or decreasing mineral density, or seal a dental site. Likewise, these compositions may be used to treat a bone defect, injury, tumour, anomalous growth, illness, or bone loss, cause the formation of mineral layers at or bellow the surface of a bone, or alter the density a bone such as for example by increasing or decreasing density. In these applications, a composition comprising one or more calcium binding peptides as described above is applied at or near the site of the affected bone or bones. Compositions comprising the calcium binding peptides disclosed herein may be used to treat calcification, calcareous lesions, or mineralized defects in tissues and organs other than bone, including arterial plaque.

"Treating" or "treatment," of a condition as used herein may refer to preventing or repairing the condition, delaying or slowing the onset or rate of development of the condition, reducing the risk of developing the condition, preventing or delaying the development of symptoms associated with the condition, reducing or ending symptoms associated with the condition, generating a complete or partial regression of the condition, or some combination thereof.

Compositions comprising the calcium binding peptides disclosed herein may be used as a means for specific attachment of desirable chemical moieties or particles to calcified surfaces, such as those of bones and teeth. Unlike current approaches to remineralization of tooth surfaces that rely on flooding the oral cavity with formulations of free or protein-bound calcium, these compounds cause calcium phosphate aggregates to specifically adhere to the tooth surface, thus increasing the local concentration of calcium and phosphate and increasing the probability that this calcium phosphate will be incorporated into demineralized regions of the tooth. Current pharmaceutical therapies for injuries or diseases of calcified tissues rely on surrounding the area of the desired surface, either directly (topically) or by systemic administration, with free solutions of the therapeutic compound of interest in the hope that some fraction will interact with the calcified surface.

Compositions comprising the calcium binding peptides disclosed herein may be used for *in situ* and *in vivo* assays for inappropriate calcification. For example, these compositions may be used to diagnose, identify, localize, or treat calcification, calcareous lesions, or mineralized defects in tissues and organs other than bone, including for example arterial plaque, kidney stones, or sesamoids. Assays currently in use for determining the presence of calcification include dye binding methods, incorporation of radioactive isotopes, X-ray transmission analysis, and quantitative chemical analysis. Each of these methods suffers from certain disadvantages. In dye binding methods, a sample is exposed to a calcium-chelating fluorescent dye such as tetracycline, calcein, or alizarin, and incorporation of the dye into the tissue of interest is visualized. Although the dyes can be introduced *in vivo*, visualization of the signal requires excision of the tissue of interest. Treatment of fixed tissue with silver ions (von Kossa staining) may also be used to identify sites of calcification, but this method cannot be applied *in vivo* and is subject to significant levels of background staining. Incorporation of radioactive isotopes such as ⁴⁵Ca provides precise and quantitative information about the localization and rate of calcification *in vivo*, but has the drawback of exposing experimental subjects to high levels of ionizing radiation. X-ray transmission analysis provides high spatial resolution and can be accomplished in live animals, but cannot uniquely identify calcium deposits among the various other features visible in an X-ray image. *In vitro* quantitative chemical analysis of calcium deposits provides robust determinations of the type and amount of mineral present, but these methods are labor intensive and result in the loss of information about the location and structure of the tissue involved.

Compositions comprising the calcium binding peptides disclosed herein may be labeled fluorescently or otherwise and utilized as an improved means of visualizing calcified regions in a tissue of interest. These peptides can be readily synthesized in high yields, and they have improved safety, toxicity, and ease of use compared to currently available methods. The sequence or composition of the peptide may be altered to change the relative affinity of the peptide for specific tissue or surface types. This will allow the peptide to discriminate between dentin, enamel, bone, and other calcified tissues or surfaces, and between healthy and diseased tissues. This property allows these compounds to be used as probes for injuries or pathological lesions in calcified tissue. The peptides may be used alone, or in conjunction with other known methods for detecting calcification. In contrast to currently available calcium-binding fluorophores, which are limited to those that can chelate calcium ions while retaining their fluorescence, the peptides described herein may be attached to any fluorophore. This greatly expands the palette of colors that can be used to label calcified surfaces, and allows precise tailoring of emission wavelengths and detection technologies to each individual experiment. By conjugating these peptides with fluorescent, colorimetric, radioactive, NMR-active, or other dyes or indicators and treating biological samples with these conjugates, it becomes possible to make quantitative observations of the extent of calcification *in situ* and *in vivo* without fixing or greatly disturbing the sample. Due to their high specificity and rapid binding rates, such conjugates may provide lower background staining than von Kossa / silver ion staining methods. The wide variety of labels that can be attached to these calcium-binding peptides offers enormous flexibility with regards to binding conditions and detection methods, which greatly increases the ease and quality with which biological, biomedical, biotechnological, environmental, and other research can be conducted.

The peptides described herein have great potential as diagnostic agents because unlike current methods of identifying injuries, infections, tumors or other lesions of calcified tissues, which rely primarily on visual or radiological observation, the peptides disclosed herein may be used to detect such events without reliance on the human eye. The DSS peptides disclosed herein have been shown to specifically target demineralized enamel and nondemineralized dentin. In particular, these peptides have exhibited the ability to preferentially bind carious tooth lesions. Further, various DSS peptide variants have exhibited the ability to target precise subportions of the tooth structure, such as for example root tip dentin, basal enamel, mantle dentin, cortical enamel, and enamel surface. Compositions comprising calcium binding peptides conjugated to various detectable moieties, such as for example fluorescent, colorimetric, radioactive, NMR-active or other dyes or indicators, may be administered to a subject to target specific portions of the tooth and to identify those portions of the tooth exhibiting demineralization or other damage. The amino acid composition of the peptides may be selected such that specific types of tissue or tissue damage may be targeted. Use of these peptides will allow for specific identification of damaged regions including those that may have been too small to see or otherwise obscured, greatly increasing the ease and accuracy of diagnosis for these lesions. Likewise, compositions comprising the calcium binding peptides disclosed herein may be used as contrast agents for X-ray, Computed Tomography, or Magnetic Resonance Imagine.

Compositions comprising the calcium binding peptides disclosed herein may be used to target therapeutic compounds to the surfaces of bones, teeth, or other calcified tissues. For example, peptides may be conjugated to antimicrobial compounds, bone and tooth development modulators, or any other compound that may be attached to the peptide, Conjugation of a therapeutic compound to one of these peptides may be used to localize the therapeutic compound to a calcified surface, leading to increased local concentration of the compound and enhanced effectiveness. By localizing the compound to a tissue of interest, these peptides will reduce the concentration of the compound needed to achieve the desired effect. In addition to improving efficacy, specific targeting of the therapeutic compound to a tissue of interest spares nontarget tissues from potentially damaging effects of the compound. The composition or length of the peptide may be adjusted to allow specific targeting to injured or diseased regions of the tissue.

Compositions comprising the calcium binding peptides disclosed herein may be used to treat a microbial infection, such as for example a bacterial infection. The peptides may be linked to an antimicrobial peptide, such as for example a 2c-4, b-34, or PL-135 peptide (SEQ ID NOs: 26, 30, and 32, respectively).

Based on the ability of the calcium binding peptides disclosed herein to selectively bind calcium or calcium phosphates, compositions comprising these peptides may be incorporated into a sensor for the detection of calcium in drinking water, wastewater, industrial solutions, foods, beverages, research applications, or any solution for which determination of the presence of calcium is desired. Likewise, these compositions may be used to control the deposition of calcium minerals in, for example, industrial, manufacturing, medical, research, household, or personal applications. Further, these compositions may be employed to determine the presence or amount of various calcium minerals in, for example, cell cultures, tissues, experimental animals, experimental human subjects, or other research applications.

The calcium binding peptides disclosed herein may be directly or indirectly linked, either covalently or noncovalently, to one or more conjugates or moieties. Such conjugates are moieties include, but are not limited to, other peptides, polypeptides, proteins, carbohydrates, nucleic acids, lipids, organic compounds, inorganic compounds, organometallic compounds, therapeutic moieties such as for example an anticancer or antimicrobial agent. Other examples of conjugates or moieties that may be linked to the calcium binding peptides disclosed herein include detectable markers such as for example fluorophores, chromophores, affinity tags, radioactive labels, or spin labels. In addition, one or more atoms within a calcium binding peptide or an attached conjugate or moiety may be replaced with a radioactive or NMR-active isotope. The linkage between a calcium binding peptide and a conjugate or moiety may occur at the amino terminus of the peptide, the carboxy terminus of the peptide, or through an internal site in the peptide. The peptide may be linked to a conjugate or moiety via an amino acid linker, such as for example a triglycine linker sequence.

Compositions comprising the calcium binding peptides disclosed herein may be administered via any method known in the art. Such methods include, but are not limited to, oral, parenteral, transdermal, aerosol, or enthral. "Oral" administration may be accomplished using a toothpaste, gel, mouthwash, mouth rinse, pill, tablet, capsule, gel, or powder. Alternatively, the compositions may be incorporated into food, chewing gum, candy, or a drink. "Parenteral" refers to a route of administration that is generally associated with injection, including infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. "Transdermal" administration may be accomplished using a topical cream, ointment, or salve, or by means of a transdermal patch. In those applications wherein the compositions are being used to treat a tooth condition or alter tooth characteristics such as mineral density, it may be administered at or near the site of the affected or target tooth Likewise, when the composition is being used to treat a bone condition or alter bone characteristics, it may be administered at or near the site of the affected or target bone.

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention. it will be understood that many variations can be made in the procedures herein described while still remaining within the bounds of the present invention. It is the intention of the inventors that such variations are included within the scope of the invention.

### Examples

### Example 1: Binding of DSS peptides to calcium hydroxyapatite:

Four DSS peptides containing two (2DSS, SEQ ID NO:12), four (4DSS, SEQ ID NO:13), six (6DSS, SEQ ID NO: 14), or eight (8DSS, SEQ ID NO:15) Asp-Ser-Ser (DSS) repeats were generated. Peptides were labeled with fluorescein, and various concentrations of the labeled peptides (0-100 µM) were mixed with a fixed amount (0.3 mg) of hydroxyapatite nanocrystals having a specific surface area of 100 m²/g (Berkeley Advanced Biomaterials, Inc.). Samples were incubated for ten minutes, followed by removal of the hydroxyapatite by centrifugation. The amount of peptide in the mixture was measured both before and after hydroxyapatite removal by spectroscopic absorbance at 480nm (the peak absorbance of the fluorescein label). The amount of peptide bound was calculated by comparing the ratio of the final (A_{f}) and initial (Aᵢ) absorbances to the initial concentration (P₀) [P_{bound}= (A_{f}/Aᵢ)P₀]. Plots were generated illustrating the amount of peptide bound per m² of hydroxyapatite surface area versus concentration of unbound peptide at equilibrium. The resulting isotherms were fit to the Langmuir isotherm (x/m=(K_{A}N_{Max}C_{eq})/(1+K_{A}C_{eq})) (Calis 1995), which describes the binding activity of a molecule in terms of a combination of binding affinity (K_{A}) and avidity (N_{Max}). K_{A} represents the affinity constant of the peptide for the hydroxyapatite surface. In this equation, x/m represents the molar amount of peptide bound per unit of hydroxyapatite surface area, N_{Max} represents the maximum surface concentration (mol/m²), and C_{eq} represents the molar concentration of unbound peptide at equilibrium. The Langmuir isotherm describes the binding of molecules to surfaces with the conditions that 1) all binding sites have the same affinity for the peptide, and 2) the peptide will form a monolayer on the surface but cannot accumulate to higher levels. The excellent fit of the Langmuir isotherm to the experimental data validates these conditions, and these affinity constants were used for comparisons between peptides. As shown in Figure 1A, the binding affinity of the various peptides for hydroxyapatite increased with an increase in the number of DSS repeats (2DSS, K_{A}=57,000 M⁻¹; 4DSS, K_{A}=94.000 M⁻¹; 8DSS, K_{A}=300,000 M⁻¹).

Several variant DSS peptides were tested to determine the effect of various amino acid alterations on binding affinity. These variant peptides included a four repeat peptide containing a longer sidechain at the first position (4ESS, SEQ. ID NO. 16), three four repeat peptides containing more sterically hindered hydroxyl groups at the second and third positions (4DTT, SEQ. ID NO. 17; 4NTT, SEQ. ID NO. 18; and 4ETT, SEQ. ID NO. 19), a four repeat peptide lacking a charged group at the first position (4NSS, SEQ. ID NO. 20), an eight repeat peptide lacking a charged group at the first position (8ASS, SEQ. ID NO. 21), and two eight repeat peptides lacking hydroxyl groups at the second and third positions (8DAA, SEQ. ID NO. 22; 8NAA, SEQ. ID NO. 23). By comparing the binding isotherms of each of these variants with that of DSS-containing peptides of the same size, it was determined that elimination of the negatively charged residue (4NSS, K_{A}=41,000 M⁻¹; 4NTT, K_{A}=18,000 M⁻¹; 8ASS, K_{A}=170,000 M⁻¹) (Figure 1B, 1C) or replacement of the serine residues with threonine or alanine (4DTT, K_{A}=161,000 M⁻¹; 4ETT, K_{A}=61.000 M⁻¹; 8DAA, K_{A}=300,000 M⁻¹) (Figure 1B, 1C) significantly decreased binding activity. Replacement of both the acidic residue at the first position and the serines led to a near-total loss of binding affinity (4NTT; 8NAA, K_{A}=20,000 M⁻¹) (Figure 1B, 1C). Replacement of the aspartic acid residue with a Glutamic acid residue caused only a slight decrease in binding affinity (4ESS, K_{A}=81,000 M⁻¹) (Figure 1B). These data suggest that both the acidic residue and the serine repeats are crucial to avidity of these peptides for hydroxyapatite surfaces. DSS was found to be the optimal repeat sequence for generating hydroxyapatite-binding activity, with all of the variant peptides showed markedly reduced binding to hydroxyapatite *in vitro.*

A partially phosphorylated four repeat DSS peptide (4DSpS, SEQ ID NO:25) was also examined and shown to have a binding affinity for hydroxyapatite similar to that of 4DSS (K_{A}=83,000 M⁻¹, versus K_{A}=94,000 M⁻¹ for 4DSS). However, the 4DS_{P}S peptide had a significantly greater number of available binding sites on the HA surface (N_{Max}=1.2x10⁻⁷ mol/m² for 4DS_{P}S versus 5.8x10⁻⁸ mol/m² for 4DSS).

### Example 2: Binding of DSS peptides to teeth:

In order to demonstrate binding of DSS peptides to biological tissue, sagitally sectioned human teeth were incubated for ten minutes in a solution of 12.5 µM 5(6) carboxyfluorescein-labeled 6DSS peptide containing 10 mM NaCl and 50 mM HEPES, pH 7.0. Control samples were prepared without peptide. Samples were rinsed after treatment and imaged by confocal laser scanning microscopy (CLSM) using blue laser illumination (excitation wavelength (λ)=488nm) and a FITC emission filter. Intense fluorescent staining indicated that 6DSS binds to the tooth surface (Figure 1D). Mock-treated control sections exhibited no fluorescence. Peptide binding was limited to the dentin (Figure 1D, light colored region, left side), with no binding visible in the enamel region (Figure 1D, dark colored region, right side).

### Example 3: Binding of DSS peptides to mineralized mouse bone marrow nodules:

Mouse bone marrow cultures were grown to confluence in DMEM+ 10% FBS, then treated continuously for three weeks with either 2.5 µM 5(6)-carboxyfluorescein-labeled 6DSS or 2.5 µM 5(6)-carboxyfluorescein-labeled peptide #3-1 (scrambled control peptide, SEQ. ID NO. 24) in aMEM+10% FBS with 50 µg/mL ascorbic acid 4 mM *β*-glycerophosphate. Cultures were imaged by fluorescence microscopy using a FITC excitation/emission filter set, and both brightfield and Fluorescence images were obtained. Strong staining was observed in the DSS-treated samples, as indicated by the light coloration shown in the central nodule mass in Figure 2B. No staining was observed in the control sample (Figure 2C, 2D), indicating that the 6DSS peptide binds specifically to mineralizing nodules in mouse bone marrow cultures.

### Example 4: Accumulation of calcium phosphate (CaHPO₄) by immobilized DSS peptides:

Streptavidin-coated polystyrene beads with an average diameter of 4 µm (Spherotech P.L.C.) were incubated with either biotin-conjugated 8DSS peptide or unconjugated biotin. Beads were washed with PBS to remove unbound peptide (or unbound biotin, in the case of the control beads) and incubated in a solution of PBS + 1 mM CaCl₂ + 1 mM NaHPO₄ for twelve days prior to imaging. As illustrated in Figure 3A, nearly all of the DSS peptide-coated beads were incorporated into large aggregates of precipitated amorphous calcium phosphate. All calcium phosphate aggregates of significant size were associated with one or more beads. By comparison, in the biotin-blocked control sample, nearly all beads were unaggregated and unassociated with precipitate (Figure 3B). Several of the peptide-coated beads became covered with more ordered layers of mineral during the experiment (representative shown in Figure 3C), while none of the uncoated/biotin blocked control beads accumulated mineral (Figure 3D).

### Example 5: Remineralization of degraded dentin surfaces with DSS peptides:

Extracted human teeth were sagitally sectioned (Accutom-50, CA-231 diamond blade) and demineralized with 19% EthyleneDiaminoTetraAcetic acid (EDTA) gel for one hour, followed by immersion in deionized water and ultrasonication to remove excess debris. Samples were treated with 12.5 µM 8DSS peptide in 50 mM HEPES buffer (pH 7.0), buffer only (no peptide), or left untreated. After one hour, samples were demineralized for 15 minutes with Quell Desensitizer (Pentron technologies, LLC), a remineralization solution consisting of aqueous solutions of calcium chloride and potassium phosphate. Samples were rinsed thoroughly prior to imaging by scanning electron microscopy (SEM). DSS-treated dentin samples accumulated a continuous layer of calcium phosphate precipitate, fully occluding the dentinal tubules (Figure 4B). Mock-treated and untreated samples exhibited much lower levels of mineral precipitate accumulation, with the dentinal tubules remaining fully exposed (Figure 4A, 4C, 4D).

### Example 6: Nucleation of hydroxyapatite on tooth enamel and dentin by DSS peptides:

To determine whether applying DSS to various preparations of tissue promoted HA nucleation, which could ultimately lead to tissue remineralization, sagittally sectioned adult human teeth (obtained after extraction during normal clinical practice) were polished using a Streurs grinding wheel, and prepared for nucleation experiments. Half of the sections were demineralized with 35% phosphoric acid for 15 minutes, then rinsed thoroughly with deionized water. The other half were left untreated. All samples were then sonicated for five minutes to remove excess debris left from cutting and grinding and/or demineralization. Samples were treated with 12.5 µM 8DSS dissolved in 50 mM HEPES buffer solution (pH 7.0), buffer solution only, or left untreated. Samples were then immersed into a simulated body fluid (SBFn), meant to accelerate nucleation of hydroxyapatite (HA) crystals, for 4 hours. Following the nucleation step, samples were immersed in a magnesium and bicarbonate free solution (SBFg) in order to allow the nucleated HA crystals to grow. Table 1 shows the composition of SBFn and SBFg solutions in comparison with blood plasma. Both solutions were adjusted to pH 6.8. Crystals were grown in order to amplify the nucleated crystals for easy visualization by SEM.

**Table 1: SBFn and SBFg Solution Composition:**

| Ionic Concentration | Na⁺ | K⁺ | Ca²⁺ | Mg²⁺ | HCO₃⁻ | Cl⁻ | HPO₄²⁻ | SO₄²⁻ |
|---|---|---|---|---|---|---|---|---|
| Blood Plasma | 142.0 | 5.0 | 2.5 | 1.5 | 27.0 | 103.0 | 1.0 | 0.5 |
| SBFn | 284.0 | 10.0 | 5.0 | 3.0 | 54.0 | 206.0 | 2.0 | 1.0 |
| SBFg | 284.0 | 4.0 | 5.0 | 0 | 0 | 294.0 | 2.0 | 0 |

The surface of the nondemineralized, untreated, and buffer treated enamel remained largely amorphous, indicating little or no crystal growth (and thus little or no nucleation) (Figure 5, upper two rows). However, significant crystal growth (indicating early and robust nucleation) was observed in demineralized enamel exposed to 8DSS peptide (Figure 5, upper two rows). This indicates that DSS peptides can specifically recognize demineralized enamel and nucleate hydroxyapatite growth on the demineralized enamel surface.

The surface of the nondemineralized dentin exhibited some degree of crystal growth in the untreated and buffer treated samples. However, the most significant growth, and thus the earliest and most robust nucleation, occurred in the sample treated with the 8DSS peptide (Figure 5, lower two rows). Whereas Example 5 demonstrated the ability of DSS peptides, in conjunction with existing remineralization regimens, to cause deposition of thick layers of calcium phosphate sufficient to occlude the dentinal tubules on demineralized dentin, these results indicate that with a slightly different treatment crystal nucleation occurs primarily on nondemineralized dentin. Thus, depending on the specific treatment regimen employed, DSS peptides can be used to deposit layers of calcium phosphate on demineralized dentin or to cause robust nucleation of hydroxyapatite crystal growth on fully mineralized surfaces. This means that DSS peptides can be used, for example, to treat tooth sensitivity due to the exposure of dentinal tubules, to remineralize mechanically debrided dentininvolved caries, or to repair fractured dentinal surfaces.

### Example 7: Tissue specificity of DSS peptide binding:

Sagitally sectioned human teeth were either demineralize by incubation with 0.5 M EDTA for 15 minutes or left nondemineralized. Samples were then incubated for ten minutes in a solution of 12.5 *µ*M 5(6)-carboyfluorescein-labeled 8DSS, 10 mM NaCl, and 50 mM HEPES, pH 7.0. Control samples were incubated without peptide. Sections were then rinsed and imaged by CLSM using identical laser and camera settings.

In the demineralized samples, DSS peptide binding was observed primarily in the enamel (Figure 6, left panel, E), with little or no binding in the dentin (Figure 6, left panel, D). Consistent with the results discussed in Example 1, the opposite pattern was observed in the nondemineralized sample, where the peptide bound primarily with the dentin and exhibited little or no binding with the enamel (Figure 6, right panel, compare D and E). The dentin-enamel junction was clearly demarcated in both the demineralized and nondemineralized samples (Figure 6, DEJ), The ability of a DSS peptide to specifically bind the enamel portion of demineralized tooth sections while showing no significant binding to nondemineralized enamel is consistent with the finding in Example 6 that DSS peptides can specifically recognize demineralized enamel and nucleate hydroxyapatite growth on the demineralized enamel surface.

### Example 8: Remineralization of bone:

In order to determine whether the remineralization results observed for tooth tissues can be extended to bone, rat femurs were obtained from sacrificed animals under a shared tissue protocol. Test samples were demineralized with 19% EDTA gel for one hour, followed by immersion in deionized water and ultrasonication to remove excess debris. Test samples were then treated with 8DSS peptide for one hour, rinsed, and half of the samples were demineralized using Quell Desensitizer (Pentron Clinical Technologies, LLC) as described in Example 5. All samples were then prepared for SEM and imaged as in Example 5. A continuous layer of mineral covering the surface of the tissue was observed in the untreated rat femur (Figure 7, upper panel). In the demineralized sample, the Haversian canals were cleanly exposed (Figure 7, middle panel). However, following treatment with 8DSS and Quell Desensitizer, the mineral surface was restored and occlusion of the canals was observed (Figure 7, lower panel). Thus, DSS peptides are able to promote remineralization of bone as well as tooth tissue.

### Example 9: Tissue specific binding of DSS peptides in human teeth:

In order to investigate the tissue specificity of DSS peptide binding to tooth tissue, sections of human teeth were exposed to DSS peptides and variants, then imaged by CLSM as described in Example 2. The peptides utilized for these experiments were 8DSS, 8ASS, 8DAA, 8NAA, 4DSS, 4ESS, 4NSS, 4DTT, 4ETT, 44NTT, and 6DSS. Sagittal sections of human teeth extracted during normal clinical practice were polished and then incubated for ten minutes in a solution of the appropriate 5(6) carboxyfluorescein-labeled peptide (12.5µM) containing 10 mM NaCl and 50 mM HEPES, pH 7.0. Control samples were prepared without peptide. Samples were washed extensively after treatment and imaged by CLSM using blue laser illumination (*λ*=488nm) and a FITC emission filter, with identical camera and microscope settings for each sample. For each section, multiple scans were collected and assembled in an automated mode to generate panels of images representing an area of 13x13 mm, sufficient in most cases to encompass the whole section. As suggested by the binding affinity results in Example 1, peptides containing the sequence (DSS)ₙ exhibited the highest levels of binding to tooth surfaces, with 6DSS and 8DSS showing the greatest levels of staining. The results of these experiments are set forth in Figure 8. 8DSS, 6DSS, and 4DSS bound primarily to the mantle dentin, with a sharply delineated dentin-enamel junction, low or no binding to the root tip dentin or the basal enamel, and no detectable binding to either the cortical enamel or the enamel surface. Significant binding was also seen to the edges of the pulp cavity. 8ASS, 4ESS, and 4NSS exhibited similar patterns, though at lower levels of binding. 8DAA exhibited an inversion of this binding pattern, with primary binding to the root tip dentin and cortical enamel, as well as to the dentin-enamel junction and the pulp cavity wall. 8DAA exhibited little or no binding to the mantle dentin, circumpulpal dentin, or basal enamel. 4DTT and 4ETT exhibited binding patterns similar to those of 4DSS and 4ESS, though at sharply reduced levels. 8NAA exhibited very little binding to any healthy tissue, but did bind somewhat to a carious lesion present in the sample (see Example 10, below). 4NTT exhibited strong binding to a fragment of periodontal bone attached to the sample, but very low levels of binding to the healthy tooth tissue. These results suggest that specific layers of the tooth can be targeted with high specificity using specific peptides.

### Example 10: Preferential binding of DSS peptides to carious lesions in teeth:

Sections of human teeth containing obvious carious lesions, which consist of demineralized enamel, were polished and exposed to 5(6)-carboxyfluorescein-labeled DSS peptides and variants using the protocol described in Example 2. The sections were washed extensively and imaged by CLSM using blue laser illumination (*λ*=488nm) and a FITC emission filter, with microscope and camera settings adjusted for each sample to optimize the signal detected from each peptide. The images were examined to identify the relative levels of peptide binding in healthy versus carious tissue. 4ESS, 4NSS, 8DAA, 8NAA, 4ETT, 4DSS, 8ASS, 8DSS, and 6DSS exhibited highly specific binding to carious lesions, with little or no binding to the surrounding healthy enamel, (Figure 9; some of these lesions are visible in Figure 8 as well). Other peptides were not tested, but based on these results binding to carious lesions is presumed. Of these peptides, 4ESS, 4NAA, 8DSS, 6DSS, 4DSS, 8DAA, and 8ASS exhibited exceptionally strong staining of carious lesions with relatively weak (often completely absent) binding of surrounding enamel. The ability of these peptides to bind carious lesions, while exhibiting little or no binding to the fully mineralized surface of healthy enamel, indicate that these peptides can be used to identify dental caries or lesion of the tooth. Given their ability to remineralize at sites of enamel degradation (Example 6), they can also be used to initiate remineralization at sites of enamel degradation such as caries or injury sites without causing inappropriate nucleation on healthy tissue surfaces.

### Example 11: Selective binding of DSS peptides to calcium phosphates:

Phosphate salts of magnesium, Calcium, manganese, cobalt, nicker, copper, and strontium were prepared by combining 100 mM sodium phosphate (pH 7.5) with 100 mM solutions of the chloride or sulfate salts of the aforementioned metal ions (MgCl₂, CaCl₂, MnCl₂, CoCl₂, NiSO₄, CuSO₄, and SrCl₂, respectively). Precipitates were collected immediately and washed twice with deionized water and dried for storage, For analysis of peptide binding, a suspension was made of each phosphate salt at a concentration of approximately 0.5% (w/v) in a solution containing 50 mM HEPES pH 7.0.10 mM NaCl, and 12.5 µM 5(6)-carboxyfluorescein-labeled 8DSS peptide. Each sample was incubated for ten minutes at room temperature, then washed twice with a solution of 50 mM HEPES pH 7.0 and 10 mM NaCl, resuspended in the same buffer, and imaged by fluorescence microscopy. Identical microscope and camera settings were used for each sample, allowing quantitative comparisons between samples. For each sample, brightfiefd and fluorescence images were collected of a single field. The intensity of fluorescent staining of the inorganic phosphate particles in each sample was assessed by measuring the pixel intensity using the GIMP (http://www.gimp.org) as follows. Using the brightfield image as a guide, areas of the fluorescence image corresponding to phosphate-salt aggregates were selected and the pixel intensities of these regions were recorded. These were compared to the intensities recorded for background regions (regions known not to contain any aggregates). These data were then expressed as the ratio of staining intensity (Iₛₜₐᵢₙ) to background intensity (I_{background}), multiplied by the absolute magnitude of the difference between the staining intensity and the background intensity (in order to correct for the high noise levels present in samples with very low levels of staining) using the equation: Relative Fluorescence=(I_{stain/}I_{background})*(Iₛₜₐₙ-I_{background}). Although a small level of staining was seen in NiHPO₄ aggregates, the highest levels of staining were seen in CaHPO₄ and the chemically similar SrHPO₄ aggregates (Figure 10). This demonstrates that the binding of DSS peptides is not a nonspecific surface adhesion phenomenon with inorganic precipitates but rather involves a highly selective interaction with calcium phosphates, even to the extent that they can distinguish between calcium and strontium phosphate precipitates.

### Example 12: Binding of DSS peptides to calcium oxalate:

Calcium oxalate was prepared using a previously described method (Wang 2006). Crystals of calcium oxalate were washed with deionized water, then resuspended in a solution containing 50 mM HEPES pH 7.0, 10 mM NaCl, and 12.5 µM 5(6)-carboxyfluorescein-labeled 8DSS peptide, The crystal suspension was incubated at room temperature for ten minutes. The crystals were then harvested by centrifugation, washed twice with a solution of 50 mM HEPES pH 7.0 and 10 mM NaCl, and visualized by fluorescence microscopy as described in Example 11. The presence of the labeled peptide led to significant staining of the calcium oxalate aggregates (Figure 11). Because calcium oxalate is the most common compound present in kidney stones (nephrolithiasis) (Coe 2005), these results suggest that DSS peptides may be used to target kidney stones in diagnostic or therapeutic applications, and that they may be able to modulate the growth of kidney stones.

### Example 13: Antimicrobial activities of DSS peptide-antimicrobial compound fusions:

In order to determine the suitability of DSS peptides to serve as targeting moieties to deliver therapeutic compounds to mineralized surfaces, peptides were synthesized containing an N-terminal (DSS)₄, (DSS)₅, or (DSS)₆ peptide, a triglycine (GGG) linker, and a 2c-4 antimicrobial peptide (SEQ ID NO:26) (J. He, unpublished). The sequences of these fusion proteins are set forth in SEQ ID NOs:27-29, respectively. The antimicrobial activity of these peptides against anaerobic planktonic bacteria was determined by a modification of a previously described assay (Qi 2005). Briefly, *Streptococcus mutans* strain UA159 cells were diluted to ∼1×10⁵ cfu/mL in Todd-Hewitt (TH) broth medium and mixed with either suspended hydroxyapatite nanocrystals (Berkeley Advanced Biomaterials, Inc., 0,03% w/v) or, for control samples, an equivalent volume of deionized water. Aliquots were transferred into 96-well plates (Fisher). Serial dilutions of the peptides were then made and added to the bacteria, The minimum inhibitory concentration (MIC) of each peptide was determined by identifying the concentration of peptide that completely inhibited bacteria growth after an incubation of approximately 24 hours, as measured by ahsorbance of cell suspensions at a wavelength of 600 nm. Peptide 2c-4 shows an MIC of 2 µM against planktonic *S*. *mutans* by itself. When conjugated to a (DSS)₄ moiety to generate peptide 4DSS-2c4, its MIC rises to 52.5 µM, a significant loss of efficacy that is unaffected by the addition of 0.03% (w/v) hydroxyapatite. However, as was shown in Example 1, the (DSS)₄ moiety shows lower affinity for hydroxyapatite than do peptides with more DSS repeats, and the high positive charge of the 2c-4 peptide may interact with the high negative charge of the (DSS)₄ moiety to inhibit activity somewhat. Nonetheless, some antimicrobial activity is retained by this peptide.

Alternatively, conjugation of a (DSS)₆ moiety to b-34 antimicrobial peptide (SEQ ID No:30) (J. He, unpublished) to generate peptide 6DSS-b-34 (SEQ ID NO:31) leads to an improvement in antimicrobial activity over that of the parent peptide (MIC= 3.1 µM for 6DSS-b-34 versus 5.6 µM for b-34). Although the addition of 0,03% HA reduces the antimicrobial activity of 6DSS-b-34 somewhat, the resulting MIC of 12.5 µM still represents considerable activity against *streptococcus mutans*. In yet another alternative, peptide PL-135 (SEQ ID NO:32) (R. Lehrer, unpublished) shows an MIC of 21 µM against planktonic S. *mutans* in medium alone. Conjugation of this peptide with a (DSS)₅ moiety to generate peptide 5DSS-PL135 (SEQ ID NO:33) reduces its antimicrobial activity against *S*. *mutans* to >170 µM in medium alone. Addition of 0.03% hydroxyapatite suspension to the medium leads to the recovery of much of this activity, reducing the MIC to 42.5 µM.

This shows that other compounds can maintain their activity when conjugated with DSS peptides. Further, this demonstrates that compounds can be readily generated that only have significant activity in the presence of a DSS peptide target, suggesting a facile means of developing compounds that are only active at calcified (bone, tooth, atc.) surfaces and are inert in other environments. Such compounds would represent a major step forward in enhancing the safety and efficacy of therapeutic approaches to mineralized tissue disorders.

### REFERENCES

1. Calis, S., et al. 1995. Pharm Res 12:1072-1076.
2. Coe, F.L., et al, 2005. J Clin Invest 115:2598-2608.
3. Cross, K,J., et al. 2005. J Pept Res 66:59-67.
4. Gu, K., et al. 2000. Eur J Oral Sci 108:35-42.
5. Hao, J., et al. 2004. Bone 34:921-932.
6. Lee, S.L., et al, 1980. Int J Pept Protein Res 16:231-240.
7. Lussi, A., et al. 1988. Arch Oral Biol 33:685-691.
8. Qi, F., et al. 2005. FEMS Microbiol Lett 251:321-326.
9. Saito, T., et al. 2000. J Bone Miner Res 15:1615-1619.
10. Veis, A., et al. 1998. Eur J Oral Sci 106 Suppl 1:234-238.
11. Wang, L., et al. 2006. Langmuir 22:7279-7285.

Further disclosed are the follwowing items:
Item 1 relates to a composition comprising one or more calcium binding peptides, wherein said calcium binding peptides each comprise the sequence (X-Y-Z)ₙ, wherein X is an amino acid selected from the group consisting of aspartic acid, glutamic acid, asparagine, alanine and glutamine, Y and Z are amino acids selected from alanine, serine, threonine, phosphoserine, and phosphothreonine and n is a number from 1 to 40, and wherein said calcium binding peptides bind calcium phosphate.
Item 2 relates to the composition of item 1, wherein said one or more calcium binding peptides have a length of about 3 to about 100 amino acids.
Item 3 relates to the composition of item 1, wherein n is a number from 2 to 8.
Item 4 relates to the composition of item 1, wherein X is aspartic acid.
Item 5 relates to the composition of item 4, wherein Y and Z are serine.
Item 6 relates to the composition of item 5, wherein said calcium binding peptides have the amino acid sequence set forth in SEQ In N0:12.
Item 7 relates to the composition of item 5, wherein said calcium binding peptides have the amino acid sequence set forth in SEQ ID N0:13.
Item 8 relates to the composition of item 5, wherein said calcium binding peptides have the amino acid sequence set forth in SEQ ID N0:14.
Item 9 relates to the composition of item 5, wherein said calcium binding peptides have the amino acid sequence set forth in SEQ ID N0:15.
Item 10 relates to the composition of item 1, wherein said calcium binding peptides further comprise a conjugate.
Item 11 relates to the composition of item 10, wherein said conjugate is selected from the group consisting of a fluorophore, chromophore, affinity tag, antigen tag, radioactive labei, or spin labei.
Item 12 relates to the composition of item 10, wherein said conjugate is selected from the group consisting of a peptide, protein, carbohydrate, nucleic acid, lipid, organic compound, inorganic compound, or organametallic compound.
Item 13 relates to the composition of item 10, wherein said conjugate is an anticancer or antimicrobial compound.
Item 14 relates to the composition of item 13, wherein said antimicrobial compound is an antimicrobial peptide.
Item 15 relates to the composition of item 14, wherein said antimicrobial peptide is linked to said one or more calcium binding peptides via an amino acid linker sequence.
Item 16 relates to a method of treating a tooth defect characterized by tooth demineralization in a subject, comprising administering the composition of item 1 to said subject, wherein said administration results in tooth remineralizsation.
Item 17 relates to a method of treating a bone defect characterized by decreased bone density in a subject, comprising administering the composition of item 1 to said subject, wherein said administration results in an increase in bone density.
Item 18 relates to a method of identifying a tooth defect characterized by tooth demineralization in a subject, comprising administering the composition of item 1 to said subject, wherein said calcium binding peptides in said composition are conjugated to a detectable marker, and wherein said calcium binding peptides preferentially bind demineralized tooth surfaces.
Item 19 relates to a method of identifying a bone defect characterized by bone demineralization in a subject, comprising administering the composition of item 1 to said subject, wherein said calcium binding peptides in said composition are conjugated to a detectable marker, and wherein said calcium binding peptides preferentially bind to demineralized bone surfaces.
Item 20 relates to a kit comprising the composition of item 1.

## Claims

1. A calcium binding peptide comprising a three amino acid repeat sequence having the formula (X-Y-Z)ₙ, wherein:
X is an amino acid selected from the group consisting of aspartic acid, glutamic acid, asparagine and glutamine;
Y and Z are amino acids selected from alanine, serine, and threonine;
n is a number from 3 to 15;
said peptide has a length of up to 100 amino acids;
said peptide is not phosphorylated; and
said peptide binds calcium phosphate.

2. The peptide of claim 1, wherein said one or more calcium binding peptides have a length of 9 to 60 amino acids.

3. The peptide according to any one of claims 1-2, wherein n is between 3 and 10.

4. The peptide according to any one of claims 1-3, wherein X is aspartic acid (Asp).

5. The peptide of claim 4, wherein Y and Z are serine.

6. The peptide according to any one of claims 1-3, wherein X-Y-Z is Glu-Thr-Thr (SEQ. ID NO. 4).

7. The peptide according to any one of claims 1-3, wherein X-Y-Z is Asn-Ser-Ser (SEQ. ID NO. 5).

8. The peptide according to any one of claims 1-3, wherein X-Y-Z is Asn-Thr-Thr (SEQ. ID NO. 6).

9. The peptide according to any one of claims 1-3, wherein X-Y-Z is Gln-Ser-Ser (SEQ. ID NO. 7).

10. The peptide according to any one of claims 1-3, wherein X-Y-Z is Gln-Thr-Thr (SEQ. ID NO:8)

11. The peptide according to any one of claims 1-3, wherein X-Y-Z is Asp-Ser-Thr (SEQ. ID NO:9), or Asp-Ala-Ala (SEQ. ID NO:10).

12. The peptide according to any one of claims 1-11, wherein said calcium binding peptides are conjugated to a detectable marker.

13. The peptide of claim 12, wherein said peptide is conjugated to a detectable marker selected from the group consisting of a fluorophore, a chromophore, an affinity tag, an antigen tag, a radioactive label, and a spin label.

14. The Peptide according to any one of claims 1-13, wherein said calcium binding peptide is conjugated to an antimicrobial peptide.

15. The peptide of claim 14, wherein said antimicrobial peptide is linked to said calcium binding peptide via an amino acid linker sequence.

16. The peptide according to any one of claims 1-11 for use in the treatment of a tooth defect **characterized by** tooth demineralization in a subject or for use in the treatment of a bone defect **characterized by** decreased bone density in a subject.

17. The peptide according to any one of claims 12-13 for use in the identification of a tooth defect **characterized by** tooth demineralization in a subject or for use in the identification of a bone defect **characterized by** bone demineralization in a subject.

## Patentansprüche

1. Kalzium-bindendes Peptid, welches eine sich wiederholende Sequenz von drei Aminosäuren umfasst, welche die Formel (X-Y-Z)ₙ hat, wobei
X eine Aminosäure, die aus der Gruppe bestehend aus Asparaginsäure, Glutaminsäure, Asparagin, und Glutamin ausgewählt ist;
Y und Z Aminosäuren sind, die aus der Gruppe bestehend aus Alanin, Serin und Threonin ausgewählt sind,
n eine Nummer ist, die sich zwischen 3 und 15 bewegt;
besagtes Peptid eine Länge von bis zu 100 Aminosäuren hat;
besagtes Peptid nicht phosphoryliert ist; und
besagtes Peptid Kalziumphosphat binden.

2. Peptid nach Anspruch 1, wobei besagte ein oder mehrere Kalzium-bindenden Peptide eine Länge von 9 bis 60 Aminosäuren haben.

3. Peptid nach einem der Ansprüche 1 bis 2, wobei n eine Nummer von 3 bis 10 ist.

4. Peptid nach einem der Ansprüche 1 bis 3, wobei X Asparaginsäure (Asp) ist.

5. Peptid nach Anspruch 4, wobei Y und Z Serin sind.

6. Peptid nach einem der Ansprüchen 1 bis 3, wobei X-Y-Z Glu-Thr-Thr (SEQ ID NO: 4) ist.

7. Peptid nach einem der Ansprüchen 1 bis 3, wobei X-Y-Z Asn-Ser-Ser (SEQ ID NO: 5) ist.

8. Peptid nach einem der Ansprüchen 1 bis 3, wobei X-Y-Z Asn-Thr-Thr (SEQ ID NO: 6) ist.

9. Peptid nach einem der Ansprüchen 1 bis 3, wobei X-Y-Z Gln-Ser-Ser (SEQ ID NO: 7) ist.

10. Peptid nach einem der Ansprüchen 1 bis 3, wobei X-Y-Z Gln-Thr-Thr (SEQ ID NO: 8) ist.

11. Peptid nach einem der Ansprüchen 1 bis 3, wobei X-Y-Z Asp-Ser-Thr (SEQ ID NO: 9), oder Asp-Ala-Ala (SEQ ID NO: 10) ist.

12. Peptid nach einem der Ansprüche 1 bis 11, wobei besagte Kalzium-bindenden Peptide an einen detektierbaren Marker konjugiert sind.

13. Peptid gemäß Anspruch 12, wobei besagtes Peptid an einen detektierbaren Marker konjugiert ist, der aus der Gruppe bestehend aus einem Fluorophor, einem Chromophor, einem Affinitäts-Tag, einem Antigen-Tag, einem radioaktiven Label, und einem Spin-Label ausgewählt ist.

14. Peptide gemäß einem der Ansprüche 1 bis 13, wobei besagtes Kalzium-bindendes Peptid an ein anti-mikrobielles Peptid konjugiert ist.

15. Peptid nach Anspruch 14, wobei besagtes anti-mikrobielles Peptid mit besagtem Kalzium-bindenden Peptid über eine Aminosäure-Linker Sequenz verknüpft ist.

16. Peptid nach einem der Ansprüche 1 bis 11, zur Verwendung in der Behandlung eines Zahn-Defekts, der durch eine Zahn-Deminaralisierung in einem Subjekt charakterisiert ist, oder zur Verwendung in der Behandlung eines Knochen-Defekts, der durch eine verminderte Knochen Dichte in einem Subjekt charakterisiert ist.

17. Peptid nach einem der Ansprüche 12 bis 13, zur Verwendung in der Identifikation eines Zahn-Defekts, der durch eine Zahn-Deminaralisierung in einem Subjekt charakterisiert ist, oder zur Verwendung in der Identifikation eines Knochen-Defekts, der durch eine Knochen-Deminaralisierung in einem Subjekt charakterisiert ist.

## Revendications

1. Peptide de liaison du calcium comprenant une séquence répétitive de trois acides aminés ayant la formule (X-Y-Z)ₙ, où:
X est un acide aminé choisi dans le groupe consistant en l'acide aspartique, l'acide glutamique, l'asparagine et la glutamine;
Y et Z sont des acides aminés choisis parmi l'alanine, la sérine, et la thréonine;
n est un nombre de 3 à 15;
ledit peptide a une longueur allant jusqu'à 100 acides aminés;
ledit peptide n'est pas phosphorylé; et
ledit peptide se lie au phosphate de calcium.

2. Peptide selon la revendication 1, dans lequel lesdits un ou plusieurs peptides de liaison du calcium ont une longueur de 9 à 60 acides aminés.

3. Peptide selon l'une quelconque des revendications 1-2, dans lequel n est entre 3 et 10.

4. Peptide selon l'une quelconque des revendications 1-3, dans lequel X est l'acide aspartique (Asp).

5. Peptide selon la revendication 4, dans lequel Y et Z sont la sérine.

6. Peptide selon l'une quelconque des revendications 1-3, dans lequel X-Y-Z est Glu-Thr-Thr (SEQ ID NO: 4).

7. Peptide selon l'une quelconque des revendications 1-3, dans lequel X-Y-Z est Asn-Ser-Ser (SEQ ID NO: 5).

8. Peptide selon l'une quelconque des revendications 1-3, dans lequel X-Y-Z est Asn-Thr-Thr (SEQ ID NO: 6).

9. Peptide selon l'une quelconque des revendications 1-3, dans lequel X-Y-Z est Gln-Ser-Ser (SEQ ID NO: 7).

10. Peptide selon l'une quelconque des revendications 1-3, dans lequel X-Y-Z est Gln-Thr-Thr (SEQ ID NO: 8).

11. Peptide selon l'une quelconque des revendications 1-3, dans lequel X-Y-Z est Asp-Ser-Thr (SEQ ID NO: 9) ou Asp-Ala-Ala Thr (SEQ ID NO:10).

12. Peptide selon l'une quelconque des revendications 1-11, dans lequel lesdits peptides de liaison du calcium sont conjugués à un marqueur détectable.

13. Peptide selon la revendications 12, dans lequel ledit peptide est conjugué à un marqueur détectable choisi dans le groupe consistant en un fluorophore, un chromophore, un marqueur d'affinité, un marqueur antigénique, un marqueur radio-actif, et un marqueur à spin.

14. Peptide selon l'une quelconque des revendications 1-13, dans lequel ledit peptide de liaison du calcium est conjugué à un peptide antimicrobien.

15. Peptide selon la revendication 14, dans lequel ledit peptide antimicrobien est lié audit peptide de liaison du calcium via une séquence d'acides aminés de liaison.

16. Peptide selon l'une quelconque des revendications 1-11 pour utilisation dans le traitement d'un défaut des dents **caractérisé par** une déminéralisation des dents chez un sujet, ou pour utilisation dans le traitement d'un défaut des os **caractérisé par** une densité osseuse amoindrie chez un sujet.

17. Peptide selon l'une quelconque des revendications 12-13, pour utilisation dans l'identification d'un défaut des dents **caractérisé par** une déminéralisation des dents chez un sujet, ou pour utilisation dans l'identification d'un défaut des os **caractérisé par** une déminéralisation des os chez un sujet.


### SEQUENCE LISTING

<110> The Regents of the University of California
<120> Calcium Binding Peptides
<130> 563-4EPT2
<140> EP 06 804 228.2
   <141> 2007-03-31
<150> US 60/722,071
   <151> 2005-09-28
<160> 39
<170> FastSEQ for Windows Version 4.0
<210> 1
   <211> 3
   <212> PRT
   <213> Artificial Sequence
<220>
   <223> human dentin phosphoprotein (DPP, phosphophoryn) DSS motif, DSS three amino acid repeat, calcium binding DSS peptide
<400> 1
   Asp Ser Ser
    1
<210> 2
   <211> 3
   <212> PRT
   <213> Artificial Sequence
<220>
   <223> calcium binding three amino acid repeat variant
<400> 2
   Glu Ser Ser
    1
<210> 3
   <211> 3
   <212> PRT
   <213> Artificial Sequence
<220>
   <223> calcium binding three amino acid repeat variant
<400> 3
   Asp Thr Thr
   1
<210> 4
   <211> 3
   <212> PRT
   <213> Artificial Sequence
<220>
   <223> calcium binding three amino acid repeat variant
<400> 4
   Glu Thr Thr
   1
<210> 5
   <211> 3
   <212> PRT
   <213> Artificial Sequence
<220>
   <223> calcium binding three amino acid repeat variant
<400> 5
   Asn Ser Ser
   1
<210> 6
   <211> 3
   <212> PRT
   <213> Artificial Sequence
<220>
   <223> calcium binding three amino acid repeat variant
<400> 6
   Asn Thr Thr
   1
<210> 7
   <211> 3
   <212> PRT
   <213> Artificial Sequence
<220>
   <223> calcium binding three amino acid repeat variant
<400> 7
   Gln Ser Ser
   1
<210> 8
   <211> 3
   <212> PRT
   <213> Artificial Sequence
<220>
   <223> calcium binding three amino acid repeat variant
<400> 8
   Gln Thr Thr
   1
<210> 9
   <211> 3
   <212> PRT
   <213> Artificial Sequence
<220>
   <223> calcium binding three amino acid repeat variant
<400> 9
   Asp Ser Thr
   1
<210> 10
   <211> 3
   <212> PRT
   <213> Artificial Sequence
<220>
   <223> calcium binding three amino acid repeat variant
<400> 10
   Asp Ala Ala
   1
<210> 11
   <211> 3
   <212> PRT
   <213> Artificial Sequence
<220>
   <223> calcium binding three amino acid repeat variant
<400> 11
   Ala Ser Thr
   1
<210> 12
   <211> 6
   <212> PRT
   <213> Artificial Sequence
<220>
   <223> calcium binding peptide containing two DSS repeats, 2DSS
<400> 12
   Asp Ser Ser Asp Ser Ser
   1 5
<210> 13
   <211> 12
   <212> PRT
   <213> Artificial Sequence
<220>
   <223> calcium binding peptide containing four DSS repeats, 4DSS, (DSS)-4
<400> 13
   Asp Ser Ser Asp Ser Ser Asp Ser Ser Asp Ser Ser
   1 5 10
<210> 14
   <211> 18
   <212> PRT
   <213> Artificial Sequence
<220>
   <223> calcium binding peptide containing six DSS repeats, 6DSS, (DSS)-6
<400> 14
   Asp Ser Ser Asp Ser Ser Asp Ser Ser Asp Ser Ser Asp Ser Ser Asp
   1 5 10 15
   Ser Ser
<210> 15
   <211> 24
   <212> PRT
   <213> Artificial Sequence
<220>
   <223> calcium binding peptide containing eight DSS repeats, 8DSS
<400> 15
   Asp Ser Ser Asp Ser Ser Asp Ser Ser Asp Ser Ser Asp Ser Ser Asp
   1 5 10 15
   Ser Ser Asp Ser Ser Asp Ser Ser
   20
<210> 16
   <211> 12
   <212> PRT
   <213> Artificial Sequence
<220>
   <223> variant DSS four repeat peptide containing longer sidechain at first position, 4ESS
<400> 16
   Glu Ser Ser Glu Ser Ser Glu Ser Ser Glu Ser Ser
   1 5 10
<210> 17
   <211> 12
   <212> PRT
   <213> Artificial Sequence
<220>
   <223> variant DSS four repeat peptide containing more sterically hindered hydroxyl groups at second and third positions, 4DTT
<400> 17
   Asp Thr Thr Asp Thr Thr Asp Thr Thr Asp Thr Thr
   1 5 10
<210> 18
   <211> 12
   <212> PRT
   <213> Artificial Sequence
<220>
   <223> variant DSS four repeat peptide containing more sterically hindered hydroxyl groups at second and third positions, 4NTT
<400> 18
   Asn Thr Thr Asn Thr Thr Asn Thr Thr Asn Thr Thr
   1 5 10
<210> 19
   <211> 12
   <212> PRT
   <213> Artificial Sequence
<220>
   <223> variant DSS four repeat peptide containing more sterically hindered hydroxyl groups at second and third positions, 4ETT
<400> 19
   Glu Thr Thr Glu Thr Thr Glu Thr Thr Glu Thr Thr
   1 5 10
<210> 20
   <211> 12
   <212> PRT
   <213> Artificial Sequence
<220>
   <223> variant DSS four repeat peptide lacking negatively charged group at first position, 4NSS
<400> 20
   Asn Ser Ser Asn Ser Ser Asn Ser Ser Asn Ser Ser
   1 5 10
<210> 21
   <211> 24
   <212> PRT
   <213> Artificial Sequence
<220>
   <223> variant DSS eight repeat peptide lacking negatively charged group at first position, 8ASS
<400> 21
   Ala Ser Ser Ala Ser Ser Ala Ser Ser Ala Ser Ser Ala Ser Ser Ala
   1 5 10 15
   Ser Ser Ala Ser Ser Ala Ser Ser
   20
<210> 22
   <211> 24
   <212> PRT
   <213> Artificial Sequence
<220>
   <223> variant DSS eight repeat peptide lacking hydroxyl group at seond and third positions, 8DAA
<400> 22
   Asp Ala Ala Asp Ala Ala Asp Ala Ala Asp Ala Ala Asp Ala Ala Asp
   1 5 10 15
   Ala Ala Asp Ala Ala Asp Ala Ala
   20
<210> 23
   <211> 24
   <212> PRT
   <213> Artificial Sequence
<220>
   <223> variant DSS eight repeat peptide lacking hydroxyl group at seond and third positions, 8NAA
<400> 23
   Asn Ala Ala Asn Ala Ala Asn Ala Ala Asn Ala Ala Asn Ala Ala Asn
   1 5 10 15
   Ala Ala Asn Ala Ala Asn Ala Ala
   20
<210> 24
   <211> 9
   <212> PRT
   <213> Artificial Sequence
<220>
   <223> scrambled control peptide #3-1
<400> 24
   Leu Ile Lys His Ile Leu His Arg Leu
   1 5
<210> 25
   <211> 12
   <212> PRT
   <213> Artificial Sequence
<220>
   <223> calcium binding peptide containing four partially phosphorylated DSS repeats, 4DS-PS
<221> MOD_RES
   <222> (2) ... (2)
   <223> phosphoserine
<221> MOD_RES
   <222> (5)...(5)
   <223> phosphoserine
<221> MOD_RES
   <222> (8)...(8)
   <223> phosphoserine
<221> MOD_RES
   <222> (11)...(11)
   <223> phosphoserine
<400> 25
   Asp Ser Ser Asp Ser Ser Asp Ser Ser Asp Ser Ser
   1 5 10
<210> 26
   <211> 7
   <212> PRT
   <213> Artificial Sequence
<220>
   <223> antimicrobial peptide 2c-4
<221> AMIDATION
   <222> (7)...(7)
   <223> phenylalaninamide
<400> 26
   Arg Trp Arg Trp Arg Trp Phe
   1 5
<210> 27
   <211> 22
   <212> PRT
   <213> Artificial Sequence
<220>
   <223> fusion protein of N-terminal (DSS)-4, triglycine (GGG) linker and 2c-4 antimicrobial peptide, peptide 4DSS-2c4
<221> AMIDATION
   <222> (22)...(22)
   <223> phenylalaninamide
<400> 27
   Asp Ser Ser Asp Ser Ser Asp Ser Ser Asp Ser Ser Gly Gly Gly Arg
    1               5                   10                  15
   Trp Arg Trp Arg Trp Phe
               20
<210> 28
   <211> 25
   <212> PRT
   <213> Artificial Sequence
<220>
   <223> fusion protein of N-terminal (DSS)-5, triglycine (GGG) linker and 2c-4 antimicrobial peptide
<221> AMIDATION
   <222> (25)...(25)
   <223> phenylalaninamide
<400> 28
   Asp Ser Ser Asp Ser Ser Asp Ser Ser Asp Ser Ser Asp Ser Ser Gly
    1               5                   10                  15
   Gly Gly Arg Trp Arg Trp Arg Trp Phe
               20                  25
<210> 29
   <211> 28
   <212> PRT
   <213> Artificial Sequence
<220>
   <223> fusion protein of N-terminal (DSS)-6, triglycine (GGG) linker and 2c-4 antimicrobial peptide
<221> AMIDATION
   <222> (28)...(28)
   <223> phenylalaninamide
<400> 29
   Asp Ser Ser Asp Ser Ser Asp Ser Ser Asp Ser Ser Asp Ser Ser Asp
    1               5                   10                  15
   Ser Ser Gly Gly Gly Arg Trp Arg Trp Arg Trp Phe
               20                  25
<210> 30
   <211> 11
   <212> PRT
   <213> Artificial Sequence
<220>
   <223> antimicrobial peptide b-34
<221> AMIDATION
   <222> (11)...(11)
   <223> phenylalaninamide
<400> 30
   Leu Lys Arg Phe Leu Lys Trp Phe Lys Arg Phe
    1               5                  10
<210> 31
   <211> 32
   <212> PRT
   <213> Artificial Sequence
<220>
   <223> fusion protein of N-terminal (DSS)-6, triglycine (GGG) linker and b-34 antimicrobial peptide, peptide 6DSS-b-34
<221> AMIDATION
   <222> (32)...(32)
   <223> phenylalaninamide
<400> 31
   Asp Ser Ser Asp Ser Ser Asp Ser Ser Asp Ser Ser Asp Ser Ser Asp
    1               5                  10                  15
   Ser Ser Gly Gly Gly Leu Lys Arg Phe Leu Lys Trp Phe Lys Arg Phe
               20                  25                  30
<210> 32
   <211> 7
   <212> PRT
   <213> Artificial Sequence
<220>
   <223> antimicrobial peptide PL-135
<221> AMIDATION
   <222> (7)...(7)
   <223> phenylalaninamide
<400> 32
   Phe His Phe His Leu His Phe
    1               5
<210> 33
   <211> 25
   <212> PRT
   <213> Artificial Sequence
<220>
   <223> fusion protein of N-terminal (DSS)-5, triglycine (GGG) linker and PL-135 antimicrobial peptide, peptide 5DSS-PL135
<221> AMIDATION
   <222> (25)...(25)
   <223> phenylalaninamide
<400> 33
   Asp Ser Ser Asp Ser Ser Asp Ser Ser Asp Ser Ser Asp Ser Ser Gly
    1               5                  10                  15
   Gly Gly Phe His Phe His Leu His Phe
               20                  25
<210> 34
   <211> 9
   <212> PRT
   <213> Artificial Sequence
<220>
   <223> calcium binding peptide containing three DSS repeats
<400> 34
   Asp Ser Ser Asp Ser Ser Asp Ser Ser
    1               5
<210> 35
   <211> 15
   <212> PRT
   <213> Artificial Sequence
<220>
   <223> calcium binding peptide containing five DSS repeats, 5DSS, (DSS)-5
<400> 35
   Asp Ser Ser Asp Ser Ser Asp Ser Ser Asp Ser Ser Asp Ser Ser
    1               5                  10                  15
<210> 36
   <211> 21
   <212> PRT
   <213> Artificial Sequence
<220>
   <223> calcium binding peptide containing seven DSS repeats
<400> 36
   Asp Ser Ser Asp Ser Ser Asp Ser Ser Asp Ser Ser Asp Ser Ser Asp
    1               5                  10                  15
   Ser Ser Asp Ser Ser
               20
<210> 37
   <211> 12
   <212> PRT
   <213> Artificial Sequence
<220>
   <223> variant DSS four repeat peptide lacking hydroxyl group at seond and third positions, 4NAA
<400> 37
   Asn Ala Ala Asn Ala Ala Asn Ala Ala Asn Ala Ala
    1               5                  10
<210> 38
   <211> 120
   <212> PRT
   <213> Artificial Sequence
<220>
   <223> (X-Y-Z)-n calcium binding peptide three amino acid repeat, where n = 1-40 and X = Asp
<221> MOD_RES
   <222> (1)...(120)
   <223> Xaa = Ala, Ser, Thr, phosphoserine or phosphothreonine
<221> MOD_RES
   <222> (4)...(120)
   <223> three amino acid repeats (Asp-Xaa-Xaa) from positions 4-120 may be present or absent
<400> 38
   Asp Xaa Xaa Asp Xaa Xaa Asp Xaa Xaa Asp Xaa Xaa Asp Xaa Xaa Asp
    1               5                  10                  15
   Xaa Xaa Asp Xaa Xaa Asp Xaa Xaa Asp Xaa Xaa Asp Xaa Xaa Asp Xaa
               20                  25                  30
   Xaa Asp Xaa Xaa Asp Xaa Xaa Asp Xaa Xaa Asp Xaa Xaa Asp Xaa Xaa
           35                  40                  45
   Asp Xaa Xaa Asp Xaa Xaa Asp Xaa Xaa Asp Xaa Xaa Asp Xaa Xaa Asp
       50                  55                  60
   Xaa Xaa Asp Xaa Xaa Asp Xaa Xaa Asp Xaa Xaa Asp Xaa Xaa Asp Xaa
   65                  70                  75                  80
   Xaa Asp Xaa Xaa Asp Xaa Xaa Asp Xaa Xaa Asp Xaa Xaa Asp Xaa Xaa
                   85                  90                  95
   Asp Xaa Xaa Asp Xaa Xaa Asp Xaa Xaa Asp Xaa Xaa Asp Xaa Xaa Asp
               100                 105                 110
   Xaa Xaa Asp Xaa Xaa Asp Xaa Xaa
           115                 120
<210> 39
   <211> 120
   <212> PRT
   <213> Artificial Sequence
<220>
   <223> (X-Y-Z)-n calcium binding peptide with three amino acid repeat, where n = 1-40, X = Asp and Y and Z = Ser
<221> MOD_RES
   <222> (4)...(120)
   <223> three amino acid repeats (Asp-Ser-Ser) from positions 4-120 may be present or absent
<400> 39
   Asp Ser Ser Asp Ser Ser Asp Ser Ser Asp Ser Ser Asp Ser Ser Asp
    1              5                   10                  15
   Ser Ser Asp Ser Ser Asp Ser Ser Asp Ser Ser Asp Ser Ser Asp Ser
               20                  25                  30
   Ser Asp Ser Ser Asp Ser Ser Asp Ser Ser Asp Ser Ser Asp Ser Ser
           35                  40                  45
   Asp Ser Ser Asp Ser Ser Asp Ser Ser Asp Ser Ser Asp Ser Ser Asp
       50                  55                  60
   Ser Ser Asp Ser Ser Asp Ser Ser Asp Ser Ser Asp Ser Ser Asp Ser
   65                  70                  75                  80
   Ser Asp Ser Ser Asp Ser Ser Asp Ser Ser Asp Ser Ser Asp Ser Ser
                   85                  90                  95
   Asp Ser Ser Asp Ser Ser Asp Ser Ser Asp Ser Ser Asp Ser Ser Asp
               100                 105                 110
   Ser Ser Asp Ser Ser Asp Ser Ser
           115                 120